(19) Europäisches Patentamt
European Patent Office
Office européen des brevets

(11) Publication number: **0 621 267 A1**

# (12) EUROPEAN PATENT APPLICATION

(21) Application number: **94200931.7**

(22) Date of filing: **06.04.94**

(51) Int. Cl.$^5$: **C07D 221/20**, C07D 491/113, A01N 43/42, A01N 43/90, //(C07D491/113,319:00,221:00)

(30) Priority: **07.04.93 EP 93105727**

(43) Date of publication of application: **26.10.94 Bulletin 94/43**

(84) Designated Contracting States: **AT BE CH DE DK ES FR GB GR IE IT LI LU MC NL PT SE**

(71) Applicant: **SHELL INTERNATIONALE RESEARCH MAATSCHAPPIJ B.V.**
**Carel van Bylandtlaan 30**
**NL-2596 HR Den Haag (NL)**

(72) Inventor: **Carter, Paul Andrew**
**Obere-Stift-Strasse 14**
**D-55218 Ingelheim (DE)**

(74) Representative: **Allam, Peter Clerk et al**
**LLOYD WISE, TREGEAR & CO.**
**Norman House**
**105-109 Strand**
**London WC2R 0AE (GB)**

(54) **Spiropiperidine derivatives and their use as fungicides.**

(57) The invention provides spiropiperidine derivatives of the general formula

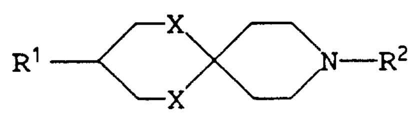

(I)

or an acid-addition salt thereof, in which $R^1$ represents a hydrogen atom or an optionally substituted alkyl, aryl or aralkyl group; $R^2$ represents a hydrogen atom or an optionally substituted alkyl, alkenyl, alkynyl, cycloalkyl, aryl, aralkyl or heterocyclyl group; and either both groups X represent $-CH_2-$ or both groups X represent -O-; processes for their preparation; compositions containing such compounds and their use as fungicides.

EP 0 621 267 A1

This invention relates to certain spiropiperidine derivatives, a process for their preparation, compositions containing such compounds and their use as fungicides.

According to the present invention there is provided a compound of the general formula

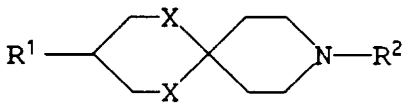

(I)

or an acid-addition salt thereof, in which $R^1$ represents a hydrogen atom or an optionally substituted alkyl, aryl or aralkyl group; $R^2$ represents a hydrogen atom or an optionally substituted alkyl, alkenyl, alkynyl, cycloalkyl, aryl, aralkyl or heterocyclyl group; and either both groups X represent $-CH_2-$ or both groups X represent -O-.

When the compounds of this invention contain an alkyl, alkenyl or alkynyl substituent group, this may be linear or branched and may contain up to 12 carbon atoms. A cycloalkyl group may contain 3 to 8, preferably 3 to 6, carbon atoms. An aryl group may be any aromatic hydrocarbon group, especially a phenyl or naphthyl group. An aralkyl group may be any alkyl group as defined above, preferably a $C_{1-6}$ alkyl and especially a $C_{1-4}$ alkyl group, substituted by an aryl group as defined above, especially a phenyl or naphthyl group. A heterocyclyl group may be any saturated or unsaturated ring system containing at least one heteroatom, 3- to 6-membered rings being preferred and 5- and 6-membered rings being especially preferred. Nitrogen-, oxygen- and sulphur-containing heterocyclic rings, such as pyridinyl, pyrimidinyl, tetrahydropyrimidinyl, pyrrolidinyl, furyl, pyranyl, morpholinyl and thienyl, are particularly preferred.

When any of the foregoing substituents are designated as being optionally substituted, the substituent groups which are optionally present may be any one or more of those customarily employed in the development of pesticidal compounds and/or the modification of such compounds to influence their structure/activity, persistence, penetration or other property. Specific examples of such substituents include, for example, halogen atoms, nitro, cyano, hydroxyl, cycloalkyl, alkyl, haloalkyl, alkoxy, haloalkoxy, amino, alkylamino, dialkylamino, formyl, alkoxycarbonyl, carboxyl, alkanoyl, alkylthio, alkylsulphinyl, alkylsulphonyl, carbamoyl, alkylamido, phenyl, phenoxy and halophenoxy groups. Typically, 0-3 substituents may be present. When any of the foregoing substituents represents or contains an alkyl substituent group, this may be linear or branched and may contain up to 12, preferably up to 6, and especially up to 4, carbon atoms. An aralkyl group may be substituted in the aryl and/or alkyl moiety.

It is preferred that $R^1$ represents a hydrogen atom or a $C_{1-12}$ alkyl or phenyl group, each group being optionally substituted by one or more substituents selected from halogen atoms, nitro, cyano, hydroxyl, $C_{3-6}$ cycloalkyl, $C_{1-4}$ alkyl, $C_{1-4}$ haloalkyl, $C_{1-4}$ alkoxy, $C_{1-4}$ haloalkoxy, amino, $C_{1-4}$ alkylamino, di-$C_{1-4}$ alkylamino, formyl, $C_{1-4}$ alkoxycarbonyl, carboxyl, phenyl and phenoxy groups.

More preferably, $R^1$ represents a hydrogen atom or a $C_{1-4}$ alkyl or phenyl group, each group being optionally substituted by one to three substituents selected from halogen atoms, nitro, cyano, $C_{1-4}$ alkyl, $C_{1-4}$ haloalkyl, $C_{1-4}$ alkoxy, $C_{1-4}$ haloalkoxy and amino groups.

It is preferred that $R^2$ represents a hydrogen atom, a $C_{1-12}$ alkyl, phenyl-$C_{1-4}$ alkyl or naphthyl-$C_{1-4}$ alkyl group or a 3- to 6-membered heterocyclic ring, each group or ring being optionally substituted by one or more substituents selected from halogen atoms, nitro, cyano, hydroxyl, $C_{3-6}$ cycloalkyl, $C_{1-4}$ alkyl, $C_{1-4}$ haloalkyl, $C_{1-4}$ alkoxy, $C_{1-4}$ haloalkoxy, amino, $C_{1-4}$ alkylamino, di-$C_{1-4}$ alkylamino, formyl, $C_{1-4}$ alkoxycarbonyl, carboxyl, phenyl and phenoxy groups.

More preferably, $R^2$ represents a hydrogen atom, a $C_{1-12}$ alkyl (especially $C_{4-12}$ alkyl) or phenyl-$C_{1-3}$ alkyl group or a 5- to 6-membered nitrogen-containing heterocyclic ring, each group or ring being optionally substituted by one to three substituents selected from halogen atoms, $C_{1-4}$ alkyl, $C_{1-4}$ haloalkyl, $C_{1-4}$ alkoxy, $C_{1-4}$ haloalkoxy, $C_{1-4}$ alkoxycarbonyl and phenyl groups.

A particularly preferred sub-group of compounds of formula I is that in which $R^1$ represents a hydrogen atom or a butyl or phenyl group; and $R^2$ represents a hydrogen atom, pentyl, hexyl, heptyl, decyl, benzyl, fluorobenzyl, chlorobenzyl, bromobenzyl, dichlorobenzyl, methylbenzyl, butylbenzyl, trifluoromethylbenzyl, methoxybenzyl, ethoxycarbonylbenzyl, phenylbenzyl, phenethyl, chlorophenethyl, phenylpropyl, dimethylpyrimidyl or dimethyltetrahydropyrimidyl group.

The compounds of formula I may form acid-addition salts with a variety of acids. However, acid-addition salts with acids such as saccharin and mineral acids, particularly hydrochloric acid, are especially preferred.

The present invention also provides a process for the preparation of a compound of formula I as defined above, or an acid-addition salt thereof, which comprises

(a) reacting a compound of the general formula

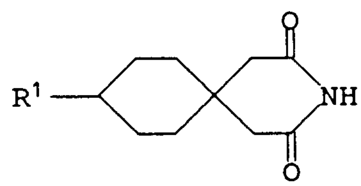

(II)

in which $R^1$ is as defined above, with a reducing agent to form a compound of formula I in which $R^2$ represents a hydrogen atom and both groups X represent $-CH_2-$; or

(b) reacting a compound of the general formula

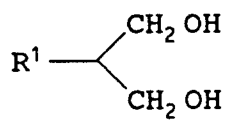

(III)

in which $R^1$ is as defined above, with a compound of the general formula

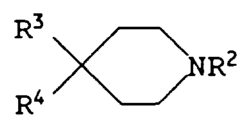

(IV)

or an acid-addition salt thereof, in which $R^2$ is as defined above and $R^3$ and $R^4$ both represent a hydroxyl group or together represent a group =O, with the proviso that, when $R^2$ represents a hydrogen atom, $R^3$ and $R^4$ both represent a hydroxyl group, in the presence of a solvent to form a compound of formula I in which both groups X represent -O-;

(c) if desired, reacting the compound formed in step (a) above or the compound formed in step (b) above in which $R^2$ represents a hydrogen atom with a compound of the general formula

$R^{2'}L$ (V)

in which $R^{2'}$ represents an optionally substituted alkyl, alkenyl, alkynyl, cycloalkyl, aryl, aralkyl or heterocyclyl group and L represents a leaving group to form a compound of formula I in which $R^2$ represents an optionally substituted alkyl, alkenyl, alkynyl, cycloalkyl, aryl, aralkyl or heterocyclyl group; and

(d) if desired, reacting the compound of formula I obtained in step (a), step (b) or step (c) above with a suitable acid to form an acid-addition salt thereof.

It is preferred that the reducing agent in step (a) is a complex metal hydride, such as lithium aluminium hydride. The process of step (a) is conveniently carried out in the presence of a solvent. Suitable solvents include ethers, particularly tetrahydrofuran, and aromatic hydrocarbons, such as toluene. The reaction is suitably carried out at a temperature in the range from 0 °C to the reflux temperature of the reaction mixture, the preferred reaction temperature being from 0 °C to 70 °C.

Suitable solvents for the process of step (b) are any solvents which facilitate the azeotropic removal of water. Such solvents include aromatic hydrocarbons, such as toluene. It is also preferred that the reaction is carried out in the presence of an acid, organic acids such as para-toluenesulphonic acid being especially preferred. The reaction is conveniently carried out at the reflux temperature of the reaction mixture.

It is preferred that the leaving group L in step (c) is a halogen, especially a chlorine, bromine or iodine, atom, an alkyl-or arylsulphonate group, such as a methylsulphonate or toluenesulphonate group, an alkylsulphonyl group, such as a methylsulphonyl group, or an alkyl- or arylthio group, such as a methylthio group. The process of step (c) is conveniently carried out in the presence of a solvent. Suitable solvents

include polar solvents such as dimethylformamide, dimethylsulphoxide, ethers (particularly tetrahydrofuran) and alcohols, such as ethanol. The reaction is suitably carried out at a temperature in the range from ambient temperature (about 15°C) to 160°C, the preferred reaction temperature being from 25°C to 150°C. It is also preferred that the reaction is carried out in the presence of a base, such as sodium or potassium carbonate.

Compounds of formula II may be conveniently prepared by reacting a compound of the general formula

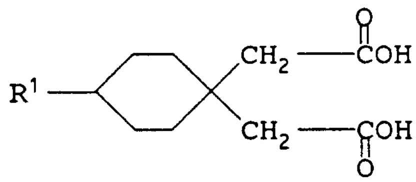

(VI)

initially with acetic anhydride and then with aqueous ammonia.

Compounds of formula VI may be prepared by reacting a compound of the general formula

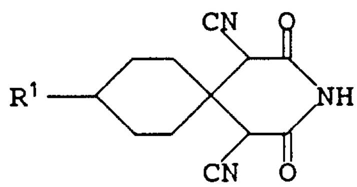

(VII)

with concentrated sulphuric acid and water.

Compounds of formula VII may be prepared by reacting a compound of the general formula

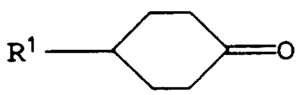

(VIII)

with a compound of formula

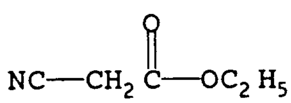

(IX)

The reaction is conveniently carried out in ethanol saturated with ammonia.

Compounds of formula III may be conveniently prepared by reacting a compound of the general formula

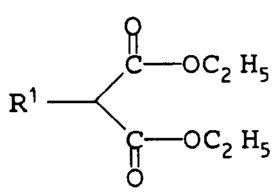

(X)

with a suitable reducing agent, such as lithium aluminium hydride, in the presence of a solvent, such as tetrahydrofuran.

Compounds of formula IV, V, VIII, IX and X are known compounds or can be prepared by processes analogous to known processes.

The compounds of general formula I have been found to have fungicidal activity. Accordingly, the invention further provides a fungicidal composition which comprises a carrier and, as active ingredient, a compound of formula I or an acid-addition salt thereof as defined above. A method of making such a composition is also provided which comprises bringing a compound of formula I as defined above, or an acid-addition salt thereof, into association with at least one carrier. Such a composition may contain a single compound or a mixture of several compounds of the present invention.

A composition according to the invention preferably contains from 0.5 to 95% by weight of active ingredient.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating fungicidal compositions may be used.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes, for example beeswax, paraffin wax, and chlorinated mineral waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example, kerosine and light mineral oils; chlorinated hydrocarbons, for example carbon tetrachloride, perchloroethylene and trichloroethane. Mixtures of different liquids are often suitable.

Fungicidal compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitol, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3-10% w of a dispersing agent and, where necessary, 0-10% w of stabiliser(s) and/or other additives such as penetrants or stickers. Dusts are usually formulated as a dust concentrate having a similar composition to that of a wettable powder but without a dispersant, and may be diluted in the field with further solid carrier to give a composition usually containing $\frac{1}{2}$-10% w of active ingredient. Granules are usually prepared to have a size between 10 and 100 BS mesh (1.676 - 0.152 mm), and may be manufactured by agglomeration or impregnation techniques. Generally, granules will contain $\frac{1}{2}$-75% w active ingredient and 0-10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 1-50% w/v active ingredient, 2-20% w/v emulsifiers and 0-20% w/v of other additives such as

stabilisers, penetrants and corrosion inhibitors. Suspension concentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick 'mayonnaise' like consistency.

The composition of the invention may also contain other ingredients, for example other compounds possessing herbicidal, insecticidal or fungicidal properties.

Of particular interest in enhancing the duration of the protective activity of the compounds of this invention is the use of a carrier which will provide a slow release of the fungicidal compounds into the environment of the plant which is to be protected. Such slow-release formulations could, for example, be inserted in the soil adjacent to the roots of a vine plant, or could include an adhesive component enabling them to be applied directly to the stem of a vine plant.

The invention still further provides the use as a fungicide of a compound of the general formula I as defined above or an acid-addition salt thereof or a composition as defined above, and a method for combating fungus at a locus, which comprises treating the locus, which may be for example plants subject to or subjected to fungal attack, seeds of such plants or the medium in which such plants are growing or are to be grown, with such a compound or composition.

The present invention is of wide applicability in the protection of crop plants against fungal attack. Typical crops which may be protected include potatoes, vines, grain crops such as wheat and barley, rice, tomatoes, broad beans and apples. The duration of protection is normally dependent on the individual compound selected, and also a variety of external factors, such as climate, whose impact is normally mitigated by the use of a suitable formulation.

The invention is further illustrated by the following examples.

Example 1

Preparation of 9-tert-butyl-3-azaspiro[5.5]undecane ($R^1$ = $-C(CH_3)_3$; $R^2$ = H; X = $-CH_2-$)

(i) Preparation of 9-tert-butyl-1,5-dicyano-2,4-dioxo-3-azaspiro[5.5]undecane

A solution of 4-tertbutylcyclohexanone (70.4g, 0.46 mol) and cyanoacetic acid ethyl ester (109 g, 0.96 mol) in ethanol (250 ml) saturated with ammonia (33 g, 1.9 mol) was allowed to stand for 19 days at 5°C. The precipitated crystals were then isolated and dried. Dissolution in hot 2N sodium hydroxide, with filtration to remove insolubles, was followed by acidification with concentrated hydrochloric acid precipitating 9-tert-butyl-1,5-dicyano-2,4-dioxo-3-azaspiro[5.5]undecane (67.7 g) as white crystals, m.pt. 228-231°C.

| Analysis | | | |
|---|---|---|---|
| Calc.:<br>Found: | C: 66.9;<br>C: 66.9; | H: 7.4;<br>H: 7.5; | N: 14.6%<br>N: 13.6% |

(ii) Preparation of 3,3-[(3-tert-butyl)pentamethylene]glutaric acid

The 9-tert-butyl-1,5-dicyano-2,4-dioxo-3-azaspiro[5.5]undecane (65.7 g, 0.23 mol) obtained in (i) above was dissolved in concentrated sulphuric acid (200 ml) at 80°C and then allowed to stand overnight. Water (170 ml) was then added in portions with stirring and the reaction mixture heated at reflux for 30 hours. The reaction mixture was then diluted with water (800 ml), cooled and filtered. The resulting black powder was decolourised with activated charcoal in acetone to give 3,3-[(3-tert-butyl)pentamethylene]glutaric acid (47.4 g) as an off-white powder, m.pt. 180-182°C.

| Analysis | | |
|---|---|---|
| Calc. : | C: 65.6 | H: 9.4% |
| Found: | C: 64.7 | H: 9.5% |

(iii) Preparation of 9-tert-butyl-2,4-dioxo-3-azaspiro[5.5]undecane

The 3,3-[(3-tert-butyl)pentamethylene]glutaric acid (45.9 g, 0.18 mol) obtained in (ii) above was heated in acetic anhydride (180 ml) at reflux for 9 hours and then evaporated to dryness. Aqueous ammonia (150 ml) was then added and the reaction mixture heated at reflux for 16 hours. The water was then distilled off and the residue heated at 230 ° C for 2 hours. Recrystallisation of the resulting solid from methanol gave 9-tert-butyl-2,4-dioxo-3-azaspiro[5.5]undecane (31.7 g) as light beige crystals, m.pt. 224-226 ° C.

| Analysis | | | |
|---|---|---|---|
| Calc.: | C: 70.9 | H: 9.8 | N: 5.9% |
| Found: | C: 71.6 | H: 9.6 | N: 5.9% |

(iv) Preparation of 9-tert-butyl-3-azaspiro[5.5]undecane

A solution of the 9-tert-butyl-2,4-dioxo-3-azaspiro[5.5]undecane (30.4 g, 0.13 mol) obtained in (iii) above in tetrahydrofuran (500 ml) was added dropwise to a suspension of lithium aluminium hydride (32 g, 0.84 mol) in tetrahydrofuran (300 ml) at 5 ° C. The reaction mixture was then heated under reflux with stirring for 40 hours, cooled and worked up with saturated aqueous sodium sulphate solution. Filtration through "Tonsil" (Registered Trade Mark: diatomaceous earth) and evaporation afforded an oil which was purified by flash column chromatography using 9:1 dichloromethane: methanol as eluant to give 9-tert-butyl-3-azaspiro[5.5]undecane (17.6 g) as white crystals, m.pt. 80-82 ° C.

| Analysis | | | |
|---|---|---|---|
| Calc.: | C: 80.3 | H: 13.0 | N: 6.7% |
| Found: | C: 79.6 | H: 12.9 | N: 6.5% |

Example 2

Preparation of 9-tert-butyl-3-(4-chlorobenzyl)-3-azaspiro[5.5]undecane

($R^1$ = $-C(CH_3)_3$; $R^2$ = 4-Cl benzyl; X = $-CH_2-$)

A mixture of the 9-tert-butyl-3-azaspiro[5.5]undecane (2.1 g, 10 mmol) obtained in Example 1 above, 4-chlorobenzyl chloride (1.6 g, 10 mmol) and potassium carbonate (1.8 g, 13 mmol) in ethanol (40 ml) was heated at reflux with stirring for 47 hours. After cooling, the solvent was evaporated and the residue partitioned between ethyl acetate and water. The organic phase was dried with magnesium sulphate and evaporated to give the crude product. Recrystallisation from isopropanol gave 9-tert-butyl-3-(4-chlorobenzyl)-3-azaspiro[5.5]undecane (1.8 g) as white crystals, m.pt. 86-87 ° C.

| Analysis | | | |
|---|---|---|---|
| Calc.: | C: 75.5 | H: 9.7 | N: 4.2% |
| Found: | C: 75.9 | H: 9.6 | N: 4.1% |

Example 3

Preparation of 3-tert-butyl-9-aza-1,5-dioxaspiro[5.5]undecane ($R^1$ = -$C(CH_3)_3$; $R^2$ = H; X = -O-)

(i) Preparation of 2-tert-butylpropan-1,3-diol

A solution of ethyl tert-butylmalonate (38 g, 0.18 mol) in tetrahydrofuran (200 ml) was added dropwise with stirring to a suspension of lithium aluminium hydride (20 g, 0.53 mol) in tetrahydrofuran (400 ml) in such a way that the temperature did not rise above 10 °C. The reaction mixture was then heated at reflux for 17 hours and then cooled and worked up with saturated aqueous sodium sulphate solution. The resulting suspension was filtered through "Tonsil" (Registered Trade Mark: diatomaceous earth) and evaporated to give slightly impure 2-tert-butylpropan-1,3-diol (17 g).

(ii) Preparation of 3-tert-butyl-9-aza-1,5-dioxaspiro[5.5]undecane

A mixture of the 2-tert-butylpropan-1,3-diol (7 g, 50 mmol) obtained in (i) above, 4-piperidone hydrate hydrochloride (8.13 g, 50 mmol) and para-toluenesulphonic acid (600 mg) in toluene (200 ml) was heated at reflux under Dean and Stark conditions for 5 hours. The reaction mixture was then cooled and water was added. The aqueous phase was made alkaline with 2N sodium hydroxide and extracted three times with chloroform. The chloroform phases were dried and evaporated to give 3-tert-butyl-9-aza-1,5-dioxaspiro[5.5]-undecane (9 g) as light yellow crystals, m.pt. 45-46 °C.

| Analysis | | | |
|---|---|---|---|
| Calc.:<br>Found: | C: 67.6<br>C: 65.1 | H: 10.9<br>H: 10.0 | N 6.6%<br>N 7.0% |

Example 4

Preparation of 3-tert-butyl-9-(4-chlorobenzyl)-9-aza-1,5-dioxaspiro[5.5]undecane

($R^1$ = -$C(CH_3)_3$; $R^2$ = 4-Cl benzyl; X = -O-)

A mixture of the 3-tert-butyl-9-aza-1,5-dioxaspiro[5.5]undecane (2 g, 9.4 mmol) obtained in Example 3 above, 4-chlorobenzyl chloride (1.5 g, 9.4 mmol) and potassium carbonate (1.7 g, 12.3 mmol) in ethanol (50 ml) was heated at reflux with stirring for 16 hours. After cooling, the solvent was evaporated and the residue partitioned between ethyl acetate and water. The organic phase was dried and evaporated to give the crude product which was purified by flash column chromatography using 4:1 petroleum ether: ethyl acetate as eluant to give 3-tert-butyl-9-(4-chlorobenzyl)-9-aza-1,5-dioxaspiro[5.5]undecane (1.7 g) as light yellow crystals, m.pt. 92-94 °C.

| Analysis | | | |
|---|---|---|---|
| Calc.:<br>Found: | C: 67.7<br>C: 67.6 | H: 8.1<br>H: 8.2 | N: 4.2%<br>N: 4.4% |

Examples 5 to 55

By processes similar to those described in Examples 1 to 4 above, further compounds according to the invention were prepared as detailed in Table I below. In this table, the compounds are identified by reference to formula I. Melting point, low resolution mass spectroscopy and C,H,N analysis data for the compounds of Examples 5 to 55 are given in Table IA below.

TABLE I

| Example No | $R^1$ | $R^2$ | X |
|---|---|---|---|
| 5 | $-C(CH_3)_3$ | benzyl | $-CH_2-$ |
| 6 | " | 4-Cl phenethyl | " |
| 7 | " | phenethyl | " |
| 8 | " | $^nC_6H_{13}$ | " |
| 9 | " | 4,6-$(CH_3)_2$-pyrimidin-2-yl | " |
| 10 (HCl Salt) | " | 4,6-$(CH_3)_2$-tetra-hydropyrimidin-2-yl | " |
| 11 | " | 2,4-$Cl_2$ benzyl | " |

TABLE I (cont'd)

| Example No | $R^1$ | $R^2$ | X |
|---|---|---|---|
| 12 | $-C(CH_3)_3$ | $^nC_5H_{11}$ | $-CH_2-$ |
| 13 | " | $^nC_7H_{15}$ | " |
| 14 | -H | -H | " |
| 15 | " | 4-Cl benzyl | " |
| 16 | " | benzyl | " |
| 17 | " | $^nC_6H_{13}$ | " |
| 18 | " | phenethyl | " |
| 19 | " | 4-Cl phenethyl | " |
| 20 | $-C(CH_3)_3$ | 4-Br benzyl | " |
| 21 | " | $4-CF_3$ benzyl | " |
| 22 | " | $4-C_6H_5$ benzyl | " |
| 23 | " | $4-CH_3$ benzyl | " |
| 24 | " | $4-C(CH_3)_3$benzyl | " |
| 25 | " | 4-F benzyl | " |
| 26 | " | $4-OCH_3$ benzyl | " |
| 27 | | $4-\overset{O}{C}-OC_2H_5$ benzyl | " |
| 28 | phenyl | -H | " |
| 29 | " | 4-Cl benzyl | " |
| 30 | " | benzyl | " |
| 31 | " | $^nC_6H_{13}$ | " |
| 32 | " | phenethyl | " |
| 33 | " | 4-Cl phenethyl | " |
| 34 | $-C(CH_3)_3$ | phenylpropyl | " |
| 35 | " | $^nC_{10}H_{21}$ | " |
| 36 | " | benzyl | -O- |
| 37 | " | $^nC_6H_{13}$ | " |
| 38 | -H | -H | " |
| 39 | " | 4-Cl benzyl | " |

TABLE I (cont'd)

| Example No | $R^1$ | $R^2$ | X |
|---|---|---|---|
| 40 | -H | $^nC_6H_{13}$ | -O- |
| 41 | phenyl | -H | " |
| 42 | " | benzyl | " |
| 43 | " | 4-Cl benzyl | " |
| 44 | " | $^nC_6H_{13}$ | " |
| 45 | $-C(CH_3)_3$ | $^nC_7H_{15}$ | " |
| 46 | " | $^nC_{10}H_{21}$ | " |
| 47 | " | phenethyl | " |
| 48 | " | 4-Cl phenethyl | " |
| 49 | " | phenylpropyl | " |
| 50 | phenyl | phenethyl | " |
| 51 | " | 4-Cl phenethyl | " |
| 52 | " | phenylpropyl | " |
| 53 | " | $^nC_7H_{15}$ | " |
| 54 | " | $^nC_{10}H_{21}$ | " |
| 55 | -H | benzyl | " |

TABLE IA

| Example No | M.Pt. °C | M+ | CHN Analysis (%) Calc.: Found: | | |
|---|---|---|---|---|---|
| 5 | 90-91 | | C:84.2<br>C:84.2 | H:11.1<br>H:11.1 | N:4.7<br>N:4.6 |
| 6 | 88-89 | | C:75.9<br>C:75.6 | H: 9.9<br>H:10.4 | N:4.0<br>N:3.8 |
| 7 | 53-54 | | C:84.3<br>C:84.5 | H:11.3<br>H:11.6 | N:4.5<br>N:4.4 |
| 8 | Oil | | C:81.8<br>C:81.3 | H:13.4<br>H:13.5 | N:4.8<br>N:4.6 |

TABLE IA (cont'd)

| Example No | M.Pt. °C | M+ | CHN Analysis (%) Calc.: Found: | | |
|---|---|---|---|---|---|
| 9 | 43-45 | | | | |
| 10 (HCl Salt) | 259-263 | | C:67.5<br>C:67.6 | H:10.8<br>H:11.2 | N:11.8<br>N:11.7 |
| 11 | 87 | | C:68.5<br>C:69.0 | H: 8.5<br>H: 8.4 | N:3.8<br>N:3.8 |
| 12 | Oil | | C:81.7<br>C:79.5 | H:13.4<br>H:13.1 | N:5.0<br>N:5.2 |
| 13 | Oil | | C:82.0<br>C:80.5 | H:13.4<br>H:13.3 | N:4.6<br>N:4.7 |
| 14 | Oil* | | C:78.4<br>C:72.1 | H:12.5<br>H:11.8 | N:9.1<br>N:8.4 |
| 15 | 71-72 | | C:73.5<br>C:73.3 | H: 8.7<br>H: 8.7 | N:5.0<br>N:5.1 |
| 16 | 46-47 | | C:83.9<br>C:82.5 | H:10.4<br>H:10.3 | N:5.8<br>N:5.8 |
| 17 | Oil | | C:80.9<br>C:80.0 | H:13.2<br>H:12.8 | N:5.9<br>N:5.9 |
| 18 | Oil | | C:84.0<br>C:81.8 | H:10.6<br>H:10.3 | N:5.4<br>N:5.3 |
| 19 | Oil | | C:74.1<br>C:70.7 | H: 9.0<br>H: 8.6 | N:4.8<br>N:4.6 |
| 20 | 95-96 | | C:66.7<br>C:66.6 | H: 8.5<br>H: 8.5 | N:3.7<br>N:3.8 |
| 21 | 79-80 | | C:71.9<br>C:71.9 | H: 8.8<br>H: 9.0 | N:3.8<br>N:3.9 |
| 22 | 134-135 | | C:86.3<br>C:86.5 | H: 9.9<br>H:10.2 | N:3.7<br>N:3.9 |

* absorbs $CO_2$ from the air

TABLE IA (cont'd)

| Example No | M.Pt. °C | M+ | CHN Analysis (%) Calc.: Found: | | |
|---|---|---|---|---|---|
| 23 | 88-89 | | C:84.3<br>C:84.0 | H:11.3<br>H:11.2 | N:4.5<br>N:4.5 |
| 24 | 75-79 | | C:84.4<br>C:84.9 | H:11.6<br>H:11.7 | N:3.9<br>N:3.9 |
| 25 | 84-85 | | C:79.5<br>C:79.3 | H:10.2<br>H:10.2 | N:4.4<br>N:4.3 |
| 26 | 87-88 | | C:80.2<br>C:81.2 | H:10.7<br>H:11.0 | N:4.3<br>N:4.2 |
| 27 | 109-110 | | C:77.6<br>C:77.5 | H:10.0<br>H:10.2 | N:3.8<br>N:3.9 |
| 28 | Oil | | C:83.8<br>C:83.5 | H:10.1<br>H:10.2 | N:6.1<br>N:7.0 |
| 29 | 97-98 | | C:78.1<br>C:78.0 | H: 8.0<br>H: 8.2 | N:4.0<br>N:4.0 |
| 30 | 84-85 | | C:86.5<br>C:86.9 | H:9.2<br>H:9.3 | N:4.4<br>N:4.5 |
| 31 | Oil | | C:84.3<br>C:83.8 | H:11.3<br>H:11.5 | N:4.5<br>N:4.9 |
| 32 | 67-68 | | C:86.4<br>C:85.5 | H: 9.4<br>H: 9.4 | N:4.2<br>N:4.8 |
| 33 | 83-84 | | C:78.3<br>C:79.5 | H: 8.2<br>H: 8.4 | N:3.8<br>N:4.0 |
| 34 | Oil | 327 | C:84.3<br>C:81.5 | H:11.4<br>H:11.2 | N:4.3<br>N:4.0 |
| 35 | Oil | 349 | C:82.4<br>C:80.1 | H:13.6<br>H:13.6 | N:4.0<br>N:4.1 |
| 36 | 62-64 | | C:75.5<br>C:75.9 | H: 9.3<br>H: 9.7 | N:4.6<br>N:4.3 |
| 37 | | | C:72.7<br>C:71.4 | H:11.9<br>H:11.7 | N:4.7<br>N:4.5 |

TABLE IA (cont'd)

| Example No | M.Pt. °C | M+ | CHN Analysis (%) Calc.: Found: | | |
|---|---|---|---|---|---|
| 38 | | | C:61.1 | H: 9.6 | N:8.9 |
| | | | C:56.6 | H: 9.2 | N:8.2 |
| 39 | | | C:63.9 | H: 7.2 | N:5.0 |
| | | | C:62.8 | H: 7.2 | N:4.8 |
| 40 | | | C:69.7 | H:11.3 | N:5.8 |
| | | | C:68.7 | H:11.2 | N:5.7 |
| 41 | 68-71 | | C:72.1 | H: 8.2 | N:6.0 |
| | | | C:70.0 | H: 8.1 | N:5.7 |
| 42 | 72-73 | | C:78.0 | H: 7.8 | N:4.3 |
| | | | C:78.1 | H: 7.8 | N:4.4 |
| 43 | 112-114 | | C:70.5 | H: 6.8 | N:3.9 |
| | | | C:69.8 | H: 6.7 | N:3.9 |
| 44 | 53-54 | | C:75.7 | H: 9.8 | N:4.4 |
| | | | C:75.5 | H: 9.9 | N:4.3 |
| 45 | | | C:73.3 | H:12.0 | N:4.5 |
| | | | C:70.0 | H:12.2 | N:3.8 |
| 46 | | | C:74.7 | H:12.3 | N:4.0 |
| | | | C:73.0 | H:12.3 | N:4.0 |
| 47 | 61-63 | | C:75.7 | H: 9.8 | N:4.4 |
| | | | C:75.2 | H:10.3 | N:4.4 |
| 48 | 92-94 | | C:68.3 | H: 8.6 | N:4.0 |
| | | | C:67.7 | H: 8.6 | N:3.9 |
| 49 | 55 | | C:76.1 | H:10.0 | N:4.2 |
| | | | C:75.5 | H:10.6 | N:4.2 |
| 50 | | | C:78.3 | H: 8.1 | N:4.2 |
| | | | C:79.0 | H: 7.9 | N:4.5 |
| 51 | | | C:71.1 | H: 7.1 | N:3.8 |
| | | | C:70.9 | H: 7.2 | N:3.8 |
| 52 | 88-90 | | C:78.6 | H: 8.3 | N:4.0 |
| | | | C:77.4 | H: 9.0 | N:4.2 |

TABLE IA (cont'd)

| Example No | M.Pt. °C | M+ | CHN Analysis (%) Calc.: Found: | | |
|---|---|---|---|---|---|
| 53 | | | C:76.1 | H:10.0 | N:4.2 |
| | | | C:73.0 | H:10.4 | N:3.9 |
| 54 | | | C:77.2 | H:10.5 | N:3.8 |
| | | | C:75.0 | H:10.1 | N:4.3 |
| 55 | | | C:72.8 | H: 8.6 | N:5.7 |
| | | | C:71.6 | H: 8.9 | N:6.0 |

Example 56

The fungicidal activity of compounds of the invention was investigated by means of the following tests.

(a) Antisporulant activity against vine downy mildew (Plasmopara viticola; PVA)

The test is a direct antisporulant one using a foliar spray. The lower surface of leaves of vine plants (cv. Cabernet Sauvignon), approximately 8cm high, are inoculated with an aqueous suspension containing 5 x $10^4$ zoosporangia/ml. The inoculated plants are kept for 24 hours at 21°C in a high humidity cabinet, then for 24 hours in a glasshouse at 20°C and 40% relative humidity. Infected leaves are sprayed on their lower surfaces with a solution of the test compound in 1:1 water/acetone containing 0.04% "TWEEN 20" (Trade Mark; a polyoxyethylene sorbitan ester surfactant). Plants are sprayed using a track sprayer equipped with 2 air-atomising nozzles. The concentration of the compound is 600 ppm and the spray volume is 750 l/ha. After drying, the plants are returned to the glasshouse at 20°C and 40% relative humidity for 96 hours and are then transferred to the high humidity cabinet for 24 hours to induce sporulation. Assessment is based on the percentage of the leaf area covered by sporulation compared with that on control leaves.

(b) Direct protectant activity against tomato late blight (Phytophthora infestans; PIP)

The test is a direct protectant one using a foliar spray. Tomato plants with two expanded leaves (cv. First in the Field) are sprayed with the test compound at a dosage of 600 ppm as described under (a). After drying, the plants are kept for 24 hours in a glasshouse at 20°C and 40% relative humidity. The upper surfaces of the leaves are then inoculated with an aqueous suspension containing 2 x $10^5$ zoosporangia/ml. The inoculated plants are kept for 24 hours at 18°C in a high humidity cabinet and then for 5 days in a growth chamber at 15°C and 80% relative humidity with 14 hours light/day. The assessment is based on the percentage of diseased leaf area compared with that on control leaves.

(c) Direct protectant activity against broad bean grey mould (Botrytis cinerea; BCB)

The test is a direct protectant one using a foliar spray. Broad bean plants (cv The Sutton) with two leaf pairs are sprayed with the test compound at a dosage of 600 ppm as described under (a). After drying, the plants are kept for 24 hours in a glasshouse at 20°C and 40% relative humidity. The upper surface of the leaves are then inoculated with an aqueous suspension containing 1 x $10^6$ conidia/ml. Plants are kept for 4 days at 22°C in a high humidity cabinet. The assessment is based on the percentage of diseased leaf area compared with that on control leaves.

(d) Direct protectant activity against barley powdery mildew (Erysiphe graminis f.sp. hordei: EGP)

The test is a direct protectant one using a foliar spray. Leaves of barley seedlings (cv. Golden Promise) at the single leaf stage are sprayed with the test compound at a dosage of 600 ppm as described under (a). After drying, the plants are kept for 24 hours in a glasshouse at 18°C and 40% relative humidity. The plants are then inoculated by dusting with mildew conidia and kept for 7 days in the glasshouse at 18°C and 40% relative humidity. Assessment is based on the percentage of leaf area covered by sporulation compared with that on leaves of control plants.

(e) Activity against barley powder mildew (Erysiphe graminis f.sp. hordei: EGT)

The test is a direct therapeutic one using a foliar spray. Leaves of barley seedlings (cv Golden

Promise) at the single leaf stage are inoculated by dusting with mildew conidia and kept in the glasshouse at 18°C and 40 relative humidity for 24 hours. Plants are then sprayed with the test comound at a dosage of 600 ppm as described under (a). After drying, plants are returned to the glasshouse at 18°C and 40% relative humidity for up to 7 days. Assessment is based on the percentage of leaf area covered by sporulation compared with that on leaves of control plants.

(f) Activity against wheat leafspot (Leptosphaeria nodorum; LN)

The test is a direct therapeutic one using a foliar spray. Wheat seedlings (cv Norman), at the single leaf stage, are inoculated with an aqueous suspension containing 1.5 x $10^6$ conidia/ml. The inoculated plants are kept for 24 hours at 20°C in a high humidity cabinet, followed by spraying with the test compound at a dosage of 600 ppm as described under (a). After drying, the plants are kept for 6-8 days in the glasshouse at 22°C and 70% relative humidity. Assessment is based on the density of lesions per leaf compared with that on leaves of control plants.

(g) Activity against wheat eyespot in-vitro (Pseudocercosporella herpotrichoides; PHI)

This test measures the in vitro activity of compounds against the fungus causing wheat eyespot. The test compound is dissolved or suspended in acetone and is added into 4 ml aliquots of half strength Potato Dextrose Broth dispensed in 25-compartment petri dishes to give a final concentration of 10 ppm test compound and 0.825% acetone. The fungal inoculum consists of mycelial fragments of P. herpotrichoides grown in half strength Potato Dextrose Broth in shaken flasks and added to the broth to provide 5 x $10^4$ mycelial fragments/ml broth. Petri dishes are incubated at 20°C for 10 days until the assessment of mycelial growth.

(h) Activity against Rhizoctonia in-vitro (Rhizoctonia solani: RSI)

The test measures the in-vitro activity of compounds against Rhizoctonia solani that causes stem and root rots. The test compound is dissolved or suspended in acetone and added into 4ml aliquots of half strength Potato Dextrose Broth dispensed in 25-compartment petri dishes to give a final concentration of 10 ppm compound and 0.825% acetone. The fungal inoculum consists of mycelial fragments of R. solani grown in half strength Potato Dextrose Broth in shaken culture flasks and added to the broth to provide 5 x $10^4$ fragments/ml broth. Petri dishes are incubated at 20°C for 10 days until the assessment of mycelial growth.

(i) Activity against apple scab in-vitro (Venturia inaequalis; VII)

This test measures the in-vitro activity of compounds against Venturia inaequalis that causes apple scab. The test compound is dissolved or suspended in acetone and added into 4ml aliquots of half strength Potato Dextrose Broth dispensed in 25-compartment petri dishes to give a final concentration of 10ppm compound and 0.825% acetone. The fungal inoculum consists of mycelial fragments and spores of V. inaequalis grown on malt agar and added to the broth to provide 5 x $10^4$ propagules/ml broth. Petri dishes are incubated at 20°C for 10 days until the assessment of mycelial growth.

The extent of disease control in all the above tests is expressed as a rating compared with either an untreated control or a diluent-sprayed-control, according to the criteria:-

0 = less than 50% disease control

1 = 50-80% disease control

2 = greater than 80% disease control

The results of these tests are set out in Table II below:-

TABLE II

| Example No. | PVA | PIP | BCB | EGP | EGT | LN | PHI | RSI | VII |
|---|---|---|---|---|---|---|---|---|---|
| 1 | | 2 | | | 2 | | 2 * | 1 * | |
| 2 | 2 | | | | 2 | 1 | 2 * | 1 * | |
| 4 | | | | | 2 | | | | |
| 5 | | | 1 | | 2 | | 1 * | | |
| 6 | | | 2 | | 2 | | 2 * | 2 * | |
| 7 | | | 1 | | 2 | 1 | 2 * | 1 * | |
| 8 | 2 | | | | 2 | 1 | | 1 * | |
| 9 | 1 | | | | 2 | | | | |
| 10 | | | | | 2 | | 1 * | 1 * | 2 * |
| 11 | | | | | 2 | | 1 * | | |
| 12 | 1 | | 1 | | 2 | 1 | 2 * | 1 * | 1 * |
| 13 | 2 | | | | 2 | | 2 * | 2 * | 1 * |
| 14 | | | 2 | | | 1 | | | |
| 15 | 2 | | | | 2 | | 1 * | | |
| 16 | | | 1 | | 1 | | | | |

**TABLE II** (cont'd)

| Example No. | PVA | PIP | BCB | EGP | EGT | LN | PHI | RSI | VII |
|---|---|---|---|---|---|---|---|---|---|
| 17 | 2 | | | | 2 | | | | |
| 18 | 2 | | 2 | | 2 | | | | |
| 19 | 2 | | | | 2 | | | | |
| 20 | | | 1 | | 2 | | 2 * | | |
| 21 | | | | | 2 | | 2 * | | 2 * |
| 22 | | | | | | | 1 | | |
| 23 | | | 2 | | 2 | | 1 | | 1 |
| 24 | | | | | 2 | | 2 | 2 | 2 |
| 25 | | | | | 2 | | 1 | 1 | 1 |
| 26 | | | | | | | 1 | 1 | 1 |
| 27 | | 1 | | | 1 | | | | |
| 28 | | | | 2 | 1 | | | | |
| 29 | | | | 2 | 2 | 1 | 2 | | 2 |
| 30 | | | | 2 | 2 | 1 | 1 | | |
| 31 | | | | 2 | 2 | 2 | 2 | | 2 |
| 32 | | | | 2 | 2 | | 2 | | 2 |
| 33 | | | | 2 | 2 | | 2 | | 2 |
| 34 | | | | 2 | 2 | | 2 | 1 | 1 |
| 35 | | | | 2 | 2 | | 2 | 1 | 2 |
| 36 | | | | | 2 | | | | |
| 37 | | | | | 2 | | | | |
| 42 | | | | | 1 | | | | |
| 43 | | | | | 2 | | 1 | | |
| 44 | | | | | 2 | | | | |
| 45 | | | | 2 | 2 | 1 | | | |
| 46 | | | | 2 | 2 | | | | |
| 47 | | | | 2 | 2 | | | | |
| 48 | | | | 2 | 2 | | | | |
| 49 | | | | 2 | 2 | 2 | | | |
| 50 | | | | 2 | 2 | | | | |

TABLE II (cont'd)

| Example No. | PVA | PIP | BCB | EGP | EGT | LN | PHI | RSI | VII |
|---|---|---|---|---|---|---|---|---|---|
| 51 | | | | 2 | 2 | 1 | | | |
| 52 | | | 2 | 2 | 2 | | | | |
| 53 | | | | 2 | 2 | | | | 1 |
| 54 | | | | 2 | 2 | | | | 1 |
| 55 | | | 1 | | | | | | |

* signifies concentration of test compound = 30 ppm

Example 56

The fungicidal activity of certain compounds of the invention was investigated using the test protocol of Example 55 (e) but at a dosage of 300 and/or 100 ppm.

The extent of disease control in this test is expressed as a rating compared with either an untreated control or a diluent-sprayed-control, according to the criteria:-

0 = < 9% disease control
1 = 9-19% " "
2 = 20-29% " "
3 = 30-39% " "
4 = 40-49% " "
5 = 50-59% " "
6 = 60-69% " "
7 = 70-79% " "
8 = 80-90% " "
9 = > 90% " "

| Example No. | Dosage (ppm) | |
|---|---|---|
| | 300 | 100 |
| 3 | 7 | 3 |
| 38 | 1 | |
| 39 | 1 | |
| 40 | 3 | 1 |
| 41 | 2 | 1 |

## Claims

**1.** A compound of the general formula

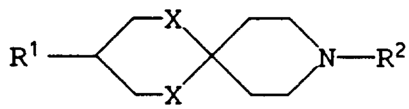

(I)

or an acid-addition salt thereof, in which $R^1$ represents a hydrogen atom or an optionally substituted alkyl, aryl or aralkyl group; $R^2$ represents a hydrogen atom or an optionally substituted alkyl, alkenyl, alkynyl, cycloalkyl, aryl, aralkyl or heterocyclyl group; and either both groups X represent $-CH_2-$ or both groups X represent -O-.

**2.** A compound according to claim 1 in which $R^1$ represents a hydrogen atom or a $C_{1-12}$ alkyl or phenyl group, each group being optionally substituted by one or more substituents selected from halogen atoms, nitro, cyano, hydroxyl, $C_{3-6}$ cycloalkyl, $C_{1-4}$ alkyl, $C_{1-4}$ haloalkyl, $C_{1-4}$ alkoxy, $C_{1-4}$ haloalkoxy, amino, $C_{1-4}$ alkylamino, di-$C_{1-4}$ alkylamino, formyl, $C_{1-4}$ alkoxycarbonyl, carboxyl, phenyl and phenoxy groups.

**3.** A compound according to claim 1 or claim 2 in which $R^2$ represents a hydrogen atom, a $C_{1-12}$ alkyl, phenyl-$C_{1-4}$ alkyl or naphthyl-$C_{1-4}$ alkyl group or a 3- to 6-membered heterocyclic ring, each group or ring being optionally substituted by one or more substituents selected from halogen atoms, nitro, cyano, hydroxyl, $C_{3-6}$ cycloalkyl, $C_{1-4}$ alkyl, $C_{1-4}$ haloalkyl, $C_{1-4}$ alkoxy, $C_{1-4}$ haloalkoxy, amino, $C_{1-4}$ alkylamino, di-$C_{1-4}$ alkylamino, formyl, $C_{1-4}$ alkoxycarbonyl, carboxyl, phenyl and phenoxy groups.

**4.** A compound according to any one of the preceding claims in which $R^1$ represents a hydrogen atom or a butyl or phenyl group; and $R^2$ represents a hydrogen atom, pentyl, hexyl, heptyl, decyl, benzyl, fluorobenzyl, chlorobenzyl, bromobenzyl, dichlorobenzyl, methylbenzyl, butylbenzyl, trifluoromethylbenzyl, methoxybenzyl, ethoxycarbonylbenzyl, phenylbenzyl, phenethyl, chlorophenethyl, phenylpropyl, dimethylpyrimidyl or dimethyltetrahydropyrimidyl group.

**5.** A compound according to claim 1 as named in any one of Examples 1 to 55.

**6.** A process for the preparation of a compound of formula I as defined in any one of the preceding claims, or an acid-addition salt thereof, which comprises

(a) reacting a compound of the general formula

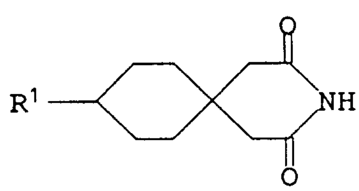

(II)

in which $R^1$ is as defined in any one of the preceding claims, with a reducing agent to form a compound of formula I in which $R^2$ represents a hydrogen atom and both groups X represent $-CH_2-$; or

(b) reacting a compound of the general formula

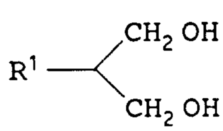

(III)

in which $R^1$ is as defined in any one of the preceding claims, with a compound of the general formula

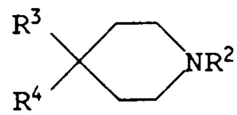

(IV)

or an acid-addition salt thereof, in which $R^2$ is as defined in any one of the preceding claims and $R^3$ and $R^4$ both represent a hydroxyl group or together represent a group =O, with the proviso that, when $R^2$ represents a hydrogen atom, $R^3$ and $R^4$ both represent a hydroxyl group, in the presence of a solvent to form a compound of formula I in which both groups X represent -O-;

(c) if desired, reacting the compound formed in step (a) above or the compound formed in step (b) above in which $R^2$ represents a hydrogen atom with a compound of the general formula

$R^{2'}L$ (V)

in which $R^{2'}$ represents an optionally substituted alkyl, alkenyl, alkynyl, cycloalkyl, aryl, aralkyl or heterocyclyl group and L represents a leaving group to form a compound of formula I in which $R^2$ represents an optionally substituted alkyl, alkenyl, alkynyl, cycloalkyl, aryl, aralkyl or heterocyclyl group; and

(d) if desired, reacting the compound of formula I obtained in step (a), step (b) or step (c) above with a suitable acid to form an acid-addition salt thereof.

**7.** A process according to claim 6 substantially as hereinbefore described and with reference to any one of Examples 1 to 4.

**8.** A compound of formula I whenever prepared by a process according to claim 6 or claim 7.

**9.** A fungicidal composition which comprises a carrier and, as active ingredient, a compound of formula I or an acid-addition salt thereof as defined in any one of claims 1 to 5 or 8.

**10.** A composition according to claim 9 which comprises at least two carriers, at least one of which is a surface-active agent.

**11.** A method of combating fungus at a locus which comprises treating the locus with a compound of formula I, or an acid-addition salt thereof, as defined in any one of claims 1 to 5 or 8 or with a compositon as defined in claim 9 or claim 10.

**12.** A method according to claim 11 in which the locus comprises plants subject to or subjected to fungal attack, seeds of such plants or the medium in which the plants are growing or are to be grown.

**13.** The use as a fungicide of a compound of formula I, or an acid-addition salt thereof as defined in any one of claims 1 to 5 or 8 or a composition as defined in claim 9 or claim 10.

European Patent Office

# PARTIAL EUROPEAN SEARCH REPORT

which under Rule 45 of the European Patent Convention shall be considered, for the purposes of subsequent proceedings, as the European search report

Application Number: EP 94 20 0931

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | CHEMICAL ABSTRACTS, 7TH COLLECTIVE INDEX, SUBJECTS 1969 , COLUMBUS, OHIO, US<br>page 2354S<br>* 3-Azaspiro[5.5]undecane and listed derivatives *<br>--- | 1-9 | C07D221/20<br>C07D491/113<br>A01N43/42<br>A01N43/90<br>//(C07D491/113, 319:00,221:00) |
| X | JOURNAL OF MEDICINAL CHEMISTRY<br>vol. 9, no. 4 , 27 June 1966 , WASHINGTON US<br>pages 555 - 558<br>J.H. ARUNDEL ET AL. 'Activity of a series of piperidines against Haemonchus contortus larvae'<br>* the whole document *<br>--- | 1-13 | |
| X | WO-A-92 10191 (ABBOTT LABORATORIES)<br>* the whole document, particularly page 8, lines 5, 6, 13, 14, 33, 34, page 9, lines 3, 4 and further examples 62, 117, 196-207, 256, 312, 330, 369, 425, 459 and 533 *<br>---<br>-/-- | 1-9 | |

TECHNICAL FIELDS SEARCHED (Int.Cl.5)

C07D
A01N

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

see sheet C

| Place of search | Date of completion of the search | Examiner |
|---|---|---|
| THE HAGUE | 12 July 1994 | Allard, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# PARTIAL EUROPEAN SEARCH REPORT

Application Number
EP 94 20 0931

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | MONATSHEFTE FüR CHEMIE<br>vol. 93, no. 5 , 1962 , WIEN AT<br>pages 1090 - 1106<br>K. STACH ET AL. 'Beiträge zur Entwicklung psychotroper Stoffe, 4. Mitt.'<br>* the whole document, particularly page 1093, table 1, Nr. 4352 and page 1100, table 6, second entry *<br>--- | 1-9 | |
| X | CHEMICAL ABSTRACTS, vol. 97, no. 23, 6 December 1982, Columbus, Ohio, US;<br>abstract no. 198165b,<br>A. CATTO ET AL.<br>'2,4-Diamino-6-piperidinyl- and 6-piperazinylpyrimidine 3-oxides, new analogs of minoxidil'<br>page 575 ;<br>* abstract *<br>& BOLL. CHIM. FARM.<br>vol. 121, no. 1 , 1982<br>pages 16 - 26<br>--- | 1-9 | TECHNICAL FIELDS SEARCHED (Int.Cl.5) |
| X | GB-A-2 000 136 (PFIZER LIMITED)<br>page 14, lines 43-50<br>--- | 1-9 | |
| X | CH-A-553 215 (ROUSSEL-UCLAF)<br>* column 7, lines 10-21 *<br>--- | 1-9 | |
| X | CH-A-469 736 (J.R. GEIGY AG)<br>* column 3, line 23, column 6, compounds j and o-q, column 7, lines 35, 36 *<br>--- | 1-9 | |
| X | US-A-3 424 755 (R. DENSS ET AL.)<br>* the whole document, particularly column 6, compound j and lines 68, 69, col. 7, ex. 2 and cpds d, e, g-q, col. 9, cpd n, col. 10, cpd q, col 13, cpd d, col 15, cpd n, col 17, cpd d *<br>----- | 1-9 | |

EP 94 20 0931

-C-

INCOMPLETE SEARCH

Claims searched completely: 11-13

Claims searched incompletely: 1-10

The number of already known compounds falling within the scope of claims 1-10 is so large that, for economical reasons, an exhaustive search report concerning these claims is not feasible (see Guidelines for examination in the EPO, B-II, 2.1). In particular, concerning compounds of formula (I), claim 1, wherein the X groups are both -CH2-, the cited documents have been limited to a single citation, i.e. Chemical Abstracts, 7th Collective Subject Index